# EUROPEAN PATENT SPECIFICATION

(11) **EP 2 245 032 B1**
(45) Date of publication and mention of the grant of the patent: **25.03.2015**
(21) Application number: 08709119.5
(22) Date of filing: 20.02.2008
(51) Int. Cl.: C07D 475/04, A61K 31/519

(54) **FOLATES, COMPOSITIONS AND USES THEREOF**
FOLATE SOWIE DEREN ZUSAMMENSETZUNGEN UND VERWENDUNGEN
FOLATES, COMPOSITIONS ET UTILISATIONS ASSOCIÉES

(43) Date of publication of application: 03.11.2010
(73) Proprietor: Gnosis S.p.A., 20033 Desio (MI) (IT)
(72) Inventor: VALOTI, Ermanno, I-20033 Desio (MI) (IT); BIANCHI, Davide, I-20033 Desio (MI) (IT); VALETTI, Marco, I-20033 Desio (MI) (IT)
(74) Representative: Mancini, Vincenzo
(86) International application number: PCT/EP2008/052037
(87) International publication number: WO 2009/103334

(56) References cited:
- EP-A- 0 409 125
- EP-A- 0 773 221
- FR-A- 2 137 186

## Description

The present invention relates to folates, compositions and uses thereof; in particular, this invention describes crystalline and amorphous compounds which are either folates or reduced folates, or the natural or unnatural isomers thereof, of at least one organic base, as well as compositions and uses thereof.

Folic acid, i.e. N-[4-[[(2-amino-1,4-dihydro-4-oxo-6-pteridinyl)methyl]amino]benzoyl]-L-glutamic acid, and folate, the anion thereof, are forms of the water-soluble vitamin B9 and the precursors of dihydrofolic and tetrahydrofolic acids, and of the respective anions thereof. They occur naturally in food, mostly as conjugates thereof, particularly in liver, kidneys, yeast, fruit and leafy green vegetables, and can also be taken as supplements. Commercially available folic acid, as the above mentioned derivatives thereof, are yet prepared synthetically.

Folic acid occurs as a yellow or yellowish-orange crystalline powder and is very slightly soluble in water and insoluble in alcohol; it is readily soluble in dilute solutions of alkali hydroxides and carbonates. Aqueous solutions of folic acid are heat sensitive and rapidly decompose in the presence of light and/or riboflavin; solutions should be therefore stored in a cool place, protected from light.

As it is well known, vitamins of the B-complex group help the body to convert carbohydrates into glucose, which is metabolized to produce energy. These vitamins are essential in the breakdown of fats and proteins and play an important role in maintaining muscle tone along the lining of the digestive tract and promoting health of the nervous system, skin, hair, eyes, mouth and liver.

It is also known that folic acid is necessary for the production and maintenance of new cells. This is especially important during periods of rapid cell division and growth such as infancy and pregnancy. Folate is needed to replicate DNA. Thus folate deficiency hinders DNA synthesis and cell division, affecting most clinically the bone marrow, a site of rapid cell turnover. Because RNA and protein synthesis are not hindered, large red blood cells, i.e. megaloblasts, are produced, resulting in macrocytic anemia, such as megaloblastic anemia (as may be seen in celiac disease) and in anemias of nutritional origin, or in pregnancy, infancy, or childhood. Accordingly, both adults and children need folate to make normal red blood cells and prevent anemia. Folate also helps prevent changes to DNA that may lead to cancer.

It is also known that folic acid derivatives such as diverse tetrahydrofolic acid derivatives can be used as drugs or as basic substance for the preparation of other derivatives. Yet, also tetrahydrofolic acid and the derivatives thereof are known to possess an extreme instability, particularly due to their susceptibility to oxidation.

In particular, 5-methyltetrahydrofolic acid has importance as a drug ingredient mainly in oncology, as concomitant therapy with methotrexate and 5-fluorouracil treatment, and in the treatment of folic acid deficiency anaemia associated with pregnancy, antibiotic therapy etc.

FR 2137186 describes water soluble alkanol ammonium salts of folic acids.

Among folates and reduced folates, the calcium salts can be mentioned as the most relatively stable derivatives: US 5,817,659 and US 6,441,168 disclose crystalline salts, preferably calcium salts, of 5-methyl-(6R,S)-, -(6S)- or - (6R)-tetrahydrofolic acid having a water of crystallization of at least one equivalent per equivalent of said acid. Calcium 5-methyltetrahydrofolate is the only folic acid derivative on the market which is able directly to penetrate the blood/brain barrier without further metabolism. Naturally occurring 5-methyltetrahydrofolic acid is solely in the S form; the R form is biochemically inactive and is excreted through the kidney.

The insolubility in water of these salts has been reported. Besides, a number of compositions for human and animal consumption, comprising either folates and/or reduced folates, are disclosed, in various forms and together with vitamins, arginine, lysine, thiamine and/or other active ingredients, for instance in US 5817659, US 5,997,915, US 6,093,703, US 6,241,996, US 6,254,904, US 6,261,600, US 6271374, US 6,440,450, US 6441168, US 6,444,218, US 6,451,360, US 6,514,973, US 6,544,944, US 6596721, US 6, 605, 646, US 6, 673, 381 US 6,808,725, US 6, 914, 073, US 6,921,754, US 6,995,158, US 2002/0094970, US 2004/0219262, US 2005/0113332, US 2006/0063768, either as a nutritional supplement or for the treatment and prevention of various diseases such as, for instance, neurological, pathopsychological, cardiovascular diseases, arthritic and inflammation conditions.

A higher chemical stability together with a desirable water-solubility which would make possible the pharmaceutical use of folates and/or reduced folates and/or the compositions comprising such compounds, without any particular precaution, is therefore still demanded.

It has been unexpectedly found that a long lasting stability as well as a peculiarly high water-solubility can be obtained by the present invention.

Preferably, the reduced folate of the present invention shows a (6R,S), (6S) or (6R) configuration and it is, in particular, a di- or tetrahydrofolate.

The folate and the reduced folate of the present invention are preferably selected from the group consisting of D-glucosamine, D-galactosamine, -folate, -dihydrofolate, - tetrahydrofolate, unsubstituted or substituted with a 5-methyl-, 5-formyl-, 10-formyl-, 5,10-methylene-, 5,10-methenyl- moiety, the compound, whenever contemplated, being in a (6R,S), (6S) or a (6R) configuration.

More preferably, the folate and reduced folate of the present invention are selected from the group consisting of D-glucosamine-folate, D-galactosamine-folate, D-glucosamine (6R,S)-tetrahydrofolate, D-glucosamine (6S)-tetrahydrofolate, D-glucosamine (6R)-tetrahydrofolate; D-galactosamine (6R,S)-tetrahydrofolate, D-galactosamine (6S)-tetrahydrofolate, D-galactosamine (6R)-tetrahydrofolate; D-glucosamine 5-methyl-(6R,S)-tetrahydrofolate, D-glucosamine 5-methyl-(6S)-tetrahydrofolate, D-glucosamine 5-methyl-(6R)-tetrahydrofolate; D-galactosamine 5-methyl-(6R,S)-tetrahydrofolate, D-galactosamine 5-methyl-(6S)-tetrahydrofolate, D-galactosamine 5-methyl-(6R)-tetrahydrofolate.

According to a preferred embodiment, the reduced folate of the present invention is in a (6S) configuration.

Still more preferably, the folate and the reduced folate are selected from the group consisting of D-glucosamine 5-methyl-(6R,S)-tetrahydrofolate, D-glucosamine 5-methyl-(6S)-tetrahydrofolate, D-glucosamine folate, D-galactosamine 5-methyl-(6R,S)-tetrahydrofolate, D-galactosamine 5-methyl-(6S)-tetrahydrofolate, D-galactosamine folate. D-glucosamine 5-methyl-(6R,S)-tetrahydrofolate, D-glucosamine 5-methyl-(6S)-tetrahydrofolate and D-glucosamine folate are the most preferred compounds of the present invention.

It is also preferred that the compound of the present invention is in an amorphous state.

Although the term "folate" is generically used in the field to collectively mean a number of chemical forms which are both structurally related and which have similar biological activity to folic acid, it will be generically referred, in the present specification, to a salt of substituted or unsubstituted folic acid and of any natural or non-natural isomers thereof and/or mixtures thereof, whereas the term "reduced folate" will generically refer to a salt of substituted or unsubstituted either dihydrofolic or tetrahydrofolic acid and of any natural or non-natural isomers thereof.

The compound of the invention shows an extraordinary long lasting chemical stability, this actually guaranteeing a purity quite unaltered, even after months, and that the titre of the correspondent folate or reduced folate moiety results substantially unchanged.

Still another peculiarity of the compound of the invention can be seen in that the counter-ion molecule of the folate and reduced folate of the compound of the invention self-evidently shows no toxicity, being already present in animal beings.

Besides to their exceptionally high chemical stability, it can also be noted that the D-glucosamine and D-galactosamine compounds of the present invention show a surprisingly complete solubility in water (even higher than 1 g/ml) which, self-evidently means an excellent bioavailability, adversely to the other folates and reduced folates hitherto known such as, for instance, alkaline and earth-alkaline salts as calcium salts.

According to another aspect, the present invention relates to a composition comprising at least one compound of the present invention.

As the skilled man will understand on the basis of the common general knowledge of the field, the composition of the invention may be formulated in various forms, either solid (f.i. tablets) or liquid (f.i. solutions), preferably in the form of a parenteral and/or oral pharmaceutical preparation, and may further comprise other inactive and/or active ingredients. Among such further ingredients, the composition of the invention may also and preferably comprise at least one of the following substances: lactose monohydrate, microcrystalline cellulose, sodium starch glycolate, stearic acid, vitamins [in particular, vitamin A, B (B1, B2, B6, B12), C, ascorbic acid, ascorbates, D (D3) E, K, PP], arginine, lysine, thiamine, essential, saturated or unsaturated, ω-3 and/or ω-6 fatty acids (preferably DHA, ARA, EPA), SAMe, cobalamin, ubiquinone, probiotics (lactobacilli, spores, yeasts), phospholipids, serine, choline, inositol, ethylendiamine, botanic extracts (blueberry, leucocyanidins, ginkgo biloba, ginseng, green tea, valerian, passion flower, camomile), melatonin, minerals, oligoelements and the like, and may be administered in an effective amount to a subject in the need thereof, depending on the needs and circumstances the case may present.

By mere way of example, the compound and/or the composition of the present invention may be administered in an amount providing for from 5% to 3000%, more preferably 5% to 200% of the subject daily folic acid requirement. In particular, the dose may amount to between 1 and 2,000 µg/day, more preferably to between 1 µg to 500 µg, most preferably to between 20 and 200 µg/day and in particular from 5 µg to 150 µg, per dose unit.

According to another aspect, the present invention discloses the use of at least one compound and/or one composition of the present invention, as above defined, for the preparation of a medicament, a food additive or a nutritional supplement, for the prevention and/or the treatment of either deficiencies or diseases positively affected by the administration of both folates and reduced folates.

By mere way of example, the compound and/or a composition of the present invention, as above defined, may be used for the preparation of a medicament, a food additive or a nutritional supplement, for the prevention and/or the treatment of neurological affection such as, for instance, subacute encephalitis associated with dementia and vacuolar myelopathies; pathopsychological, vascular and cardiovascular such as, for instance premature occlusive arterial disease, severe vascular disease in infancy and childhood, progressive arterial stenosis, intermittent claudication, renovascular hypertension, ischemic cerebrovascular disease, premature retinal artery and retinal vein occlusion, cerebral occlusive arterial disease, occlusive peripheral arterial disease, premature death due to thromboembolic disease and/or ischemic heart disease; autoimmune diseases, such as, for instance, psoriasis, celiac disease, arthritic and inflammation conditions; megaloblastic anaemia due to folate deficiency, intestinal malabsorption, for reducing a female's risk of having a miscarriage and/or of having a fetus with a neural tube defect, a cleft lip defect, and/or a cleft palate defect, for maintaining and/or normalizing the homocysteine level and/or metabolism; alterations of the synthesis and/or the functioning and/or the changes of DNA and RNA and the alterations of cell synthesis; depressive illnesses.

The compound of the invention can be prepared by simply applying the common general knowledge of the field, as the skilled man would easily understand; however, as a non-limitative example, the compound of the invention can be prepared by adding the desired substituted or unsubstituted folic or reduced folic acid to an aqueous solution containing D-glucosamine or D-galactosamine, preferably maintained under nitrogen and stirring, the base being in a molar amount of about 200 to 300% of the acid.

As a consequence, the desired acid results to be entirely dissolved and an homogeneous and clear solution, having a pH between about 6.3 and 8.0 and containing the desired folate or reduced folate, is thereby obtained.

The salts obtained from D-glucosamine or D-galactosamine with desired substituted or unsubstituted folate or reduced folate, in a molar amount of 200%, are completely water-soluble and can be self-evidently collected very easily, as the skilled man would promptly realize, by simply applying the common general knowledge of the field, for instance and alternatively by directly freeze-drying the desired folate or reduced folate solution obtained, or by spray-drying the desired folate or reduced folate solution obtained.

It can be advantageously noted that, regardless of the way of recovering carried out, the desired folate or reduced folate of the present invention is obtained in an approximatively quantitative amount.

The following examples illustrate the invention without limiting it.

### EXAMPLE 1

Preparation of D-glucosamine 5-methyl-(6R,S)-tetrahydrofolate 4.60 g (10 mmol) of 5-methyl-(6R,S)-tetrahydrofolic acid were added portion-wise and completely dissolved in 30 ml of an aqueous solution of D-glucosamine (3.58 g, 20 mmol) stirred under nitrogen. The resulting solution (pH 6.53) was freeze-dried, obtaining 8.72 g of the title product.

### Analytical data:

HPLC titre in 5-methyl-(6R,S)-tetrahydrofolic acid: calculated 56.18% (on the dry product); found 55.22% (98.3% of the theoretical value);
Specific rotation [α]²⁰_{D} = +54.2° (C = 1 in water)
NMR (D₂O): 7.45 (d, 2H); 6.55 (d, 2H); 5.20 (bs, 2H); 4. 0 5 (m, H); 3.70-3.40 (m, 7H); 3.38-3.00 (m, 6H); 2.99-2.70 (m, 4H); 2.40 (s, 3H); 2.15-2.05 (m, 2H); 2.05-1.90 (m, 1H); 1.90-1.75 (m, 1H).

### EXAMPLE 2

Preparation of D-glucosamine 5-methyl-(6R,S)-tetrahydrofolate 9.19 g (20 mmol) of 5-methyl-(6R,S)-tetrahydrofolic acid were added portion-wise and completely dissolved in 60 ml of an aqueous solution of D-glucosamine (7.12 g, 40 mmol) stirred under nitrogen. The resulting solution (pH 6.53) was spraydried, obtaining 16.9 g of the title product.

### Analytical data:

HPLC titre in 5-methyl-(6R,S)-tetrahydrofolic acid: calculated 56.18% (on the dry product); found 55.13 (98.2 % of the theoretical value);
Specific rotation [α]²⁰_{D} = +54.0 (C = 1 in water)
NMR (D₂O): 7.45 (d, 2H); 6.55 (d, 2H); 5.20 (bs, 2H); 4. 0 5 (m, H); 3.70-3.40 (m, 7H); 3.38-3.00 (m, 6H); 2.99-2.70 (m, 4H); 2.40 (s, 3H); 2.15-2.05 (m, 2H); 2.05-1.90 (m, 1H); 1.90-1.75 (m, 1H).

### EXAMPLE 3

Preparation of D-glucosamine 5-methyl-(6S)-tetrahydrofolate 4.60 g (10 mmol) of 5-methyl-(6S)-tetrahydrofolic acid, obtained by the resolution of the corresponding (6R,S)-5-methyltetraidrofolic acid, were added portion-wise and completely dissolved in 30 ml of an aqueous solution of D-glucosamine (3.58 g, 20 mmol) stirred under nitrogen. The resulting solution (pH 6.53) was freeze-dried, obtaining 8.72 g of the title product.

### Analytical data:

HPLC titre in 5-methyl-(6S)-tetrahydrofolic acid: calculated 56.18% (on the dry product); found 55.7% (99.1% of the theoretical value);
Specific rotation [α]²⁰_{D} = +42.6° (C = 1 in water)
NMR (D₂O): 7.45 (d, 2H); 6.55 (d, 2H); 5.20 (bs, 2H); 4. 0 5 (m, H); 3.70-3.40 (m, 7H); 3.38-3.00 (m, 6H); 2.99-2.70 (m, 4H); 2.40 (s, 3H); 2.15-2.05 (m, 2H); 2.05-1.90 (m, 1H); 1.90-1.75 (m, 1H).

### EXAMPLE 4

### Preparation of D-glucosamine L-folate

4.41 g (10 mmol) of L-folic acid were added portion-wise and completely dissolved in 40 ml of an aqueous solution of D-glucosamine (3.58 g, 20 mmol) stirred under nitrogen. The resulting solution (pH 6.53) was freeze-dried obtaining 7.95 g of the title product.

### Analytical data:

HPLC titre in L-folic acid: calculated 55.2% (on the dry product); found 54.6 (99.0% of the theoretical value);
Specific rotation [α]²⁰_{D} = +44.5° (C = 1 in water)
NMR (D₂O): 8.42 (s, 1H) ; 7.45 (d, 2H) ; 6.42 (d, 2H) ; 5.20 (bs, 2H); 4.25 (s, 2H); 4.05 (m, 1H); 3.75-3.45 (m, 6H); 3.35-3.20 (m, 2H); 3.15-3.00 (m, 2H); 2.15-2.05 (m, 2H); 2.05-1.90 (m, 1H); 1.90-1.75 (m, 1H).

### EXAMPLE 5

### Preparation of D-galactosamine 5-methyl-(6R,S)-tetrahydrofolate

4.60 g (10 mmol) of 5-methyl-(6R,S)-tetrahydrofolic acid were added portion-wise and completely dissolved in 30 ml of an aqueous solution of D-galactosamine (3.58 g, 20 mmol) stirred under nitrogen. The resulting solution (pH 6.53) was freeze-dried, obtaining 8.72 g of the title product.

### Analytical data:

HPLC titre in 5-methyl-(6R,S)-tetrahydrofolic acid: calculated 56.18% (on the dry product); found 55.5% (98.8% of the theoretical value);
Specific rotation [α]²⁰_{D} = +51.43° (C = 1 in water)
NMR (D₂O): 7.45 (d, 2H); 6.55 (d, 2H); 5.20 (bs, 2H); 4. 15 (m, H); 3.70-3.35 (m, 7H); 3.30-2.85 (m, 10H); 2.40 (s, 3H); 2.15-2.05 (m, 2H); 2.05-1.90 (m, 1H); 1.90-1.75 (m, 1H).

### EXAMPLE 6

Preparation of D-galactosamine 5-methyl-(6S)-tetrahydrofolate 4.60 g (10 mmol) of 5-methyl-(6S)-tetrahydrofolic acid, obtained by the resolution of the corresponding (6R,S)-5-methyltetraidrofolic acid, were added portion-wise and completely dissolved in 30 ml of an aqueous solution of D-galactosamine (3.58 g, 20 mmol) stirred under nitrogen. The resulting solution (pH 6.53) was freeze-dried, obtaining 8.72 g of the title product.

### Analytical data:

HPLC titre in 5-methyl-(6R,S)-tetrahydrofolic acid: calculated 56.18% (on the dry product); found 55.5% (98.8% of the theoretical value);
Specific rotation [α]²⁰_{D} = +66.5° (C = 1 in water)
NMR (D₂O): 7.45 (d, 2H); 6.55 (d, 2H); 5.20 (bs, 2H); 4.15 (m, H); 3.70-3.35 (m, 7H); 3.30-2.85 (m, 10H); 2.40 (s, 3H); 2.15-2.05 (m, 2H); 2.05-1.90 (m, 1H); 1.90-1.75 (m, 1H).

### EXAMPLE 7

### Preparation of D-galactosamine L-folate

4.41 g (10 mmol) of L-folic acid were added portion-wise and completely dissolved in 40 ml of an aqueous solution of D-galactosamine (3.58 g, 20 mmol) stirred under nitrogen. The resulting solution (pH 6.53) was freeze-dried obtaining 7.95 g of the title product.

### Analytical data:

HPLC titre in L-folic acid: calculated 55.2% (on the dry product); found 54.7 (99.1% of the theoretical value);
Specific rotation [α]²⁰_{D} = +49.77 (C = 1 in water)
NMR (D₂O): 8.42 (s, 1H); 7.45 (d, 2H); 6.42 (d, 2H); 5.20 (bs, 2H); 4.25 (s, 2H); 4.05 (m, 1H); 3.75-3.45 (m, 6H); 3.35-3.20 (m, 2H); 3.15-3.00 (m, 2H); 2.15-2.05 (m, 2H); 2.05-1.90 (m, 1H); 1.90-1.75 (m, 1H).

### STABILITY

The stability of the compounds listed in the following table, under powder form and in sealed aluminium foil bag, was tested, keeping the samples in airtight container, protected from light and measuring the purity and the titre thereof after 6 and 12 months.

| Compound | Starting values | | After 6 months | | After 12 months | |
|---|---|---|---|---|---|---|
| | Purity | Titre | Purity | Titre | Purity | Titre |
| D-GLU folate | 99.1 | 54.6 | 98.8 | 54.3 | 98.7 | 54.0 |
| D-GAL folate | 99.2 | 54.8 | 99.1 | 54.5 | 98.7 | 54.2 |
| D-GLU 5-MTHF | 99.1 | 55.2 | 98.9 | 55.0 | 99.0 | 54.8 |
| D-GLU (6S)-5-MTHF | 99.3 | 55.7 | 99.0 | 55.4 | 98.5 | 55.1 |
| D-GAL (6R,S)-5-MTHF | 99.1 | 55.5 | 98.9 | 55.2 | 98.7 | 55.3 |

| | | | | | | |
|---|---|---|---|---|---|---|
| D-GLU = D-glucosamine; D-GAL = D-galactosamine | | | | | | |

As a comparison, it can be noted that crystalline (6R,S)-, (6S)- and (6R)-tetrahydrofolic acid calcium salts, as disclosed in US 5,817,659 (Example 1), showed a titre decrease of about 2%, after nine months whereas the such decrease, as reported in US 6,271,374 (Example 1) for crystalline (6S)- and (6R)-tetrahydrofolic acid calcium salts, amounts to about 7% after twelve months. As to crystalline 5-methyl-(6S)-tetrahydrofolic acid calcium salts, US 6,441,168 (Example 1) reports instead a titre decrease amounting to about 1% after twelve months.

In view of the data illustrated in the table above, an expiration date of twelve months can be safely established for the compound of the present invention, further noting a titre decrease lower than 1% after twelve months. Any of the compounds listed in the above table showed a high stability since both their purity and titre, even after twelve months, resulted to fall well within the required specifications. In particular, the stability of the acid moiety was observed by HPLC, detecting the purity and titre thereof.

### BIOAVAILABILITY

The biological tests were carried out on 12 mice weighing 200-300 g, administering to each of them one 3 mg capsule containing the dose of the selected compound, as below reported, dispersed in lactose.

The haematic values of the selected compound were subsequently evaluated after regular time intervals (0, 30, 60, 95, 120 min.).

The following compounds, D-glucosamine folate (60 µg), D-glucosamine [6R,S]5-methyltetrahydrofolate (61 µg), D-glucosamine [6S]5- methyltetrahydrofolate (61 µg),D-galactosamine folate (60 µg), D-galactosamine [6R,S]5-methyltetrahydrofolate (61 µg), D-galactosamine [6S]5-methyltetrahydrofolate (61 µg), were tested and compared to the following ones, amorphous folic acid calcium salt (38 µg), amorphous [6R,S]5-methyltetrahydrofolic acid calcium salt (39 µg), amorphous [6S]-5-methyltetrahydrofolic acid calcium salt (39 µg), crystalline folic acid calcium salt (39 µg), crystalline [6R,S]-5-methyltetrahydrofolic acid calcium salt (39 µg), crystalline [6S]-5-methyltetrahydrofolic acid calcium salt (39 µg).

The biological tests showed that, by administering the compounds of the present invention, the haematic levels resulted about 20% higher than the values found administering to the animals an analogous dose of the corresponding folate and reduced folate calcium salts.

Further, it has been noted a slight and yet significant increase of the bioavailability (about 10%) by administering amorphous calcium salts in comparison with crystalline calcium salts, as illustrated in the following table.

| time (h) | D-Glu F (amorphous) (nmol/l) | D-Glu 5-MTHF (amorphous) (nmol/l) | D-Glu (6S)-5-MTHF (amorphous) (nmol/l) | Ca 5-MTHF (crystalline) (nmol/l) (comparative) | Ca 5-MTHF (amorphous) (nmol/l) (comparative) |
|---|---|---|---|---|---|
| | | | | | |
| 0,5 | 75,3 ± 4.3 | 83,2 ± 4.9 | 83,7 ± 5.2 | 63,2 ± 4.5 | 68,2 ± 5.1 |
| 1 | 79,8 ± 5.2 | 86.,5 ± 4.9 | 86,1 ± 5.0 | 69.0 ± 6.1 | 73,2 ± 5.9 |
| 1,5 | 78,4 ± 4.3 | 83,8 ± 4.3 | 83,7 ± 4.3 | 70,5 ± 4.3 | 75,2 ± 4.3 |
| 2 | 76,8 ± 4.3 | 82,8 ± 4.3 | 83 ± 4.3 | 70,1 ± 4.3 | 74,1 ± 4.3 |

| | | | | | |
|---|---|---|---|---|---|
| D-Glu = D-glucosamine; D-Gal = D-galactosamine; F = folate | | | | | |

It is therefore evident how the high water-solubility of the D-glucosamine and D-galactosamine compounds of the present invention positively and significantly affects the absorption thereof, thus enhancing the bioavailability of all the molecule active moieties.

## Claims

1. A crystalline or amorphous compound which is a folate or a reduced folate of D-glucosamine, D-galactosamine, - folate, -dihydrofolate, -tetrahydrofolate, unsubstituted or substituted with a 5-methyl-, 5-formyl-, 10-formyl-, 5,10-methylene-, 5,10-methenyl- moiety, the compound, whenever contemplated, being in a (6R,S), (6S) or a (6R) configuration.

2. The compound according to claim 1, wherein the folate and reduced folate are selected from the group consisting of D-glucosamine-folate, D-galactosamine-folate, D-glucosamine (6R,S)-tetrahydrofolate, D-glucosamine (6S)-tetrahydrofolate, D-glucosamine (6R)-tetrahydrofolate; D-galactosamine (6R,S)-tetrahydrofolate, D-galactosamine (6S)-tetrahydrofolate, D-galactosamine (6R)-tetrahydrofolate; D-glucosamine 5-methyl-(6R,S)-tetrahydrofolate, D-glucosamine 5-methyl-(6S)-tetrahydrofolate, D-glucosamine 5-methyl-(6R)-tetrahydrofolate; D-galactosamine 5-methyl-(6R,S)-tetrahydrofolate, D-galactosamine 5-methyl-(6S)-tetrahydrofolate, D-galactosamine 5-methyl-(6R)-tetrahydrofolate.

3. The compound according to claim 1 or 2, wherein the folate and the reduced folate are selected from the group consisting of D-glucosamine 5-methyl-(6R,S)-tetrahydrofolate, D-glucosamine 5-methyl-(6S)-tetrahydrofolate, D-glucosamine folate, D-galactosamine 5-methyl-(6R,S)-tetrahydrofolate, D-galactosamine 5-methyl-(6S)-tetrahydrofolate, D-galactosamine folate.

4. The compound according to any of the previous claims, which is D-glucosamine 5-methyl-(6R,S)-tetrahydrofolate or D-glucosamine 5-methyl-(6S)-tetrahydrofolate.

5. The compound according to any of claims 1-4, which is D-glucosamine folate.

6. The compound according to any of the previous claims, wherein the reduced folate is in a (6S) configuration.

7. The compound according to any of the previous claims, which is in an amorphous state.

8. The compound according to any of the previous claims, wherein the reduced folate is D-glucosamine 5-methyl-(6S)-tetrahydrofolate or D-galactosamine 5-methyl-(6S)-tetrahydrofolate.

9. The compound according to any of the previous claims, which is D-glucosamine 5-methyl-(6S)-tetrahydrofolate.

10. A composition comprising at least one compound according to any of the previous claims.

11. A composition according to the previous claim wherein the compound is a crystalline or amorphous compound which is a folate or a reduced folate, of D-glucosamine and D-galactosamine and wherein it further comprises at least one of the following substances: lactose monohydrate, microcrystalline cellulose, sodium starch glycolate, stearic acid, vitamins, ascorbic acid, ascorbates, arginine, lysine, thiamine, essential, saturated or unsaturated, ω-3 and/or ω-6 fatty acids, SAMe, cobalamin, ubiquinone, probiotics, phospholipids, serine, choline, inositol, ethylendiamine, botanic extracts, melatonin, minerals, oligoelements.

12. A composition according to claim 10 or 11, wherein the folate and reduced folate are selected from the group consisting of D-glucosamine-folate, D-galactosamine-folate, D-glucosamine (6R,S)-tetrahydrofolate, D-glucosamine (6S)-tetrahydrofolate, D-glucosamine (6R)-tetrahydrofolate; D-galactosamine (6R,S)-tetrahydrofolate, D-galactosamine (6S)-tetrahydrofolate, D-galactosamine (6R)-tetrahydrofolate; D- glucosamine 5-methyl-(6R,S)-tetrahydrofolate, D-glucosamine 5-methyl-(6S)-tetrahydrofolate, D-glucosamine 5-methyl-(6R)-tetrahydrofolate; D-galactosamine 5-methyl-(6R,S)-tetrahydrofolate, D-galactosamine 5-methyl-(6S)-tetrahydrofolate, D-galactosamine 5-methyl-(6R)-tetrahydrofolate.

13. A composition according to any of claims 10-12, wherein the folate and the reduced folate are selected from the group consisting of D-glucosamine 5-methyl-(6R,S)-tetrahydrofolate, D-glucosamine 5-methyl-(6S)-tetrahydrofolate, D-glucosamine folate, D-galactosamine 5-methyl-(6R,S)-tetrahydrofolate, D-galactosamine 5-methyl-(6S)-tetrahydrofolate, D-galactosamine folate.

14. A composition according to any of claims 10-13, wherein the compound is D-glucosamine 5-methyl-(6R,S)-tetrahydrofolate or D-glucosamine 5-methyl-(6S)-tetrahydrofolate.

15. A composition according to any of claims 10-14, wherein the compound is D-glucosamine folate.

16. A composition according to any of claims 10-15, wherein vitamins, fatty acids, probiotics and botanic extracts are selected -respectively- from the following groups consisting of: vitamin A, B, C, D, E, K, PP; DHA, ARA, EPA; lactobacilli, spores, yeasts; blueberry, leucocyanidins, ginkgo biloba, ginseng, green tea, valerian, passion flower, camomile.

17. A composition according to any of claims 10-16, wherein vitamin B is either vitamin B1, B2, B6 or B12 and vitamin D is vitamin D3.

18. Use of at least one compound and/or one composition according to any of the previous claims, for the preparation of a medicament, a food additive or a nutritional supplement, for the prevention and/or the treatment of either deficiencies or diseases positively affected by the administration of both folates and reduced folates selected from neurological affection such as, for instance, subacute encephalitis associated with dementia and vacuolar myelopathies; pathopsychological, vascular and cardiovascular such as, for instance premature occlusive arterial disease, severe vascular disease in infancy and childhood, progressive arterial stenosis, intermittent claudication, renovascular hypertension, ischemic cerebrovascular disease, premature retinal artery and retinal vein occlusion, cerebral occlusive arterial disease, occlusive peripheral arterial disease, premature death due to thromboembolic disease and/or ischemic heart disease; autoimmune diseases, such as, for instance, psoriasis, celiac disease, arthritic and inflammation conditions; megaloblastic anaemia due to folate deficiency, intestinal malabsorption, for reducing a female's risk of having a miscarriage and/or of having a fetus with a neural tube defect, a cleft lip defect, and/or a cleft palate defect, for maintaining and/or normalizing the homocysteine level and/or metabolism; alterations of the synthesis and/or the functioning and/or the changes of DNA and RNA and the alterations of cell synthesis; depressive illnesses.

## Patentansprüche

1. Kristalline oder amorphe Verbindung, die ein Folat oder ein reduziertes Folat von D-Glucosamin, D-Galactosamin, -Folat, -Dihydrofolat, -Tetrahydrofolat, unsubstituiert oder substituiert mit einer 5-Methyl-, 5-Formyl-, 10-Formyl-, 5,10-Methylen-, 5,10-Methenyl-Einheit, ist, wobei die Verbindung, wann immer betrachtet, in einer (6R,S)-, (6S)- oder einer (6R)-Konfiguration ist.

2. Verbindung gemäß Anspruch 1, wobei das Folat und das reduzierte Folat ausgewählt sind aus der Gruppe, bestehend aus D-Glucosaminfolat, D-Galactosaminfolat, D-Glucosamin-(6R,S)-tetrahydrofolat, D-Glucosamin-(6S)-tetrahydrofolat, D-Glucosamin-(6R)-tetrahydrofolat; D-Galactosamin-(6R,S)-tetrahydrofolat, D-Galactosamin-(6S)-tetrahydrofolat, D-Galactosamin-(6R)-tetrahydrofolat; D-Glucosamin-5-methyl-(6R,S)-tetrahydrofolat, D-Glucosamin-5-methyl-(6S)-tetrahydrofolat, D-Glucosamin-5-methyl-(6R)-tetrahydrofolat; D-Galactosamin-5-methyl-(6R,S)-tetrahydrofolat, D-Galactosamin-5-methyl-(6S)-tetrahydrofolat, D-Galactosamin-5-methyl-(6R)-tetrahydrofolat.

3. Verbindung gemäß Anspruch 1 oder 2, wobei das Folat und das reduzierte Folat ausgewählt sind aus der Gruppe, bestehend aus D-Glucosamin-5-methyl-(6R,S)-tetrahydrofolat, D-Glucosamin-5-methyl-(6S)-tetrahydrofolat, D-Glucosaminfolat, D-Galactosamin-5-methyl-(6R,S)-tetrahydrofolat, D-Galactosamin-5-methyl-(6S)-tetrahydrofolat, D-Galactosaminfolat.

4. Verbindung gemäß einem der vorangehenden Ansprüche, die D-Glucosamin-5-methyl-(6R,S)-tetrahydrofolat oder D-Glucosamin-5-methyl-(6S)-tetrahydrofolat ist.

5. Verbindung gemäß einem der Ansprüche 1-4, die D-Glucosaminfolat ist.

6. Verbindung gemäß einem der vorangehenden Ansprüche, wobei das reduzierte Folat in einer (6S)-Konfiguration vorliegt.

7. Verbindung gemäß einem der vorangehenden Ansprüche, die in einem amorphen Zustand vorliegt.

8. Verbindung gemäß einem der vorangehenden Ansprüche, wobei das reduzierte Folat D-Glucosamin-5-methyl-(6S)-tetrahydrofolat oder D-Galactosamin-5-methyl-(6S)-tetrahydrofolat ist.

9. Verbindung gemäß einem der vorangehenden Ansprüche, die D-Glucosamin-5-methyl-(6S)-tetrahydrofolat ist.

10. Zusammensetzung, die mindestens eine Verbindung gemäß einem der vorangehenden Ansprüche umfasst.

11. Zusammensetzung gemäß dem vorangehenden Anspruch, wobei die Verbindung eine kristalline oder amorphe Verbindung, die ein Folat oder ein reduziertes Folat von D-Glucosamin und D-Galactosamin ist, und wobei sie weiterhin mindestens eine der folgenden Substanzen umfasst: Lactosemonohydrat, mikrokristalline Zellulose, Natriumstärkeglycolat, Stearinsäure, Vitamine, Ascorbinsäure, Ascorbate, Arginin, Lysin, Thiamin, essentielle, gesättigte oder ungesättigte, ω-3- und/oder ω-6-Fettsäuren, SAMe, Cobalamin, Ubichinon, Probiotika, Phospholipide, Serin, Cholin, Inositol, Ethylendiamin, botanische Extrakte, Melatonin, Minerale, Spurenelemente.

12. Zusammensetzung gemäß Anspruch 10 oder 11, wobei das Folat und das reduzierte Folat ausgewählt sind aus der Gruppe, bestehend aus D-Glucosaminfolat, D-Galactosaminfolat, D-Glucosamin-(6R,S)-tetrahydrofolat, D-Glucosamin-(6S)-tetrahydrofolat, D-Glucosamin-(6R)-tetrahydrofolat; D-Galactosamin-(6R,S)-tetrahydrofolat, D-Galactosamin-(6S)-tetrahydrofolat, D-Galactosamin-(6R)-tetrahydrofolat; D-Glucosamin-5-methyl-(6R,S)-tetrahydrofolat, D-Glucosamin-5-methyl-(6S)-tetrahydrofolat, D-Glucosamin-5-methyl-(6R)-tetrahydrofolat; D-Galactosamin-5-methyl-(6R,S)-tetrahydrofolat, D-Galactosamin-5-methyl-(6S)-tetrahydrofolat, D-Galactosamin-5-methyl-(6R)-tetrahydrofolat.

13. Zusammensetzung gemäß einem der Ansprüche 10-12, wobei das Folat und das reduzierte Folat ausgewählt sind aus der Gruppe, bestehend aus D-Glucosamin-5-methyl-(6R,S)-tetrahydrofolat, D-Glucosamin-5-methyl-(6S)-tetrahydrofolat, D-Glucosaminfolat, D-Galactosamin-5-methyl-(6R,S)-tetrahydrofolat, D-Galactosamin-5-methyl-(6S)-tetrahydrofolat, D-Galactosaminfolat.

14. Zusammensetzung gemäß einem der Ansprüche 10-13, wobei die Verbindung D-Glucosamin-5-methyl-(6R,S)-tetrahydrofolat oder D-Glucosamin-5-methyl-(6S)-tetrahydrofolat ist.

15. Zusammensetzung gemäß einem der Ansprüche 10-14, wobei die Verbindung D-Glucosaminfolat ist.

16. Zusammensetzung gemäß einem der Ansprüche 10-15, wobei Vitamine, Fettsäuren, Probiotika und botanische Extrakte jeweils ausgewählt sind aus den folgenden Gruppen, bestehend aus: Vitamin A, B, C, D, E, K, PP; DHA, ARA, EPA; Lactobazillen, Sporen, Hefen; Blaubeere, Leukocyanidinen, Ginkgo biloba, Ginseng, grünem Tee, Baldrian, Passionsblume, Kamille.

17. Zusammensetzung gemäß einem der Ansprüche 10-16, wobei Vitamin B entweder Vitamin B1, B2, B6 oder B12 ist und Vitamin D Vitamin D3 ist.

18. Verwendung mindestens einer Verbindung und/oder einer Zusammensetzung gemäß einem der vorangehenden Ansprüche für die Herstellung eines Medikaments, eines Nahrungsmittelzusatzes oder eines Nahrungsergänzungsmittels, für die Prävention und/oder Behandlung von entweder Mängeln oder Erkrankungen, die durch die Verabreichung von sowohl Folaten als auch reduzierten Folaten positiv beeinflusst werden, ausgewählt aus neurologischer Erkrankung, wie beispielsweise subakuter Enzephalitis assoziiert mit Demenz und vakuolärer Myelopathie; pathopsychologischer, vaskulärer und kardiovaskulärer Erkrankung, wie beispielsweise vorzeitige arterielle Verschlusskrankheit, schwere vaskuläre Erkrankung im Säuglingsalter und der Kindheit, progressiver arterieller Stenose, Claudicatio intermittens, renovaskulärer Hypertonie, ischämischer zerebrovaskulärer Erkrankung, vorzeitiger Okklusion von Retinalarterie und Retinalvene, zerebraler arterieller Verschlusskrankheit, peripherer arterieller Verschlusskrankheit, vorzeitigem Tod aufgrund von thromboembolischer Erkrankung und/oder ischämischer Herzerkrankung; Autoimmunerkrankungen, wie beispielsweise Psoriasis, Zöliakie, arthritischen und inflammatorischen Zuständen; megaloblastischer Anämie aufgrund von Folatmangel, intestinaler Malabsorption, zur Verringerung des Risikos einer Frau, eine Fehlgeburt zu haben und/oder einen Fötus mit einem Neuralrohrdefekt, einem Lippenspaltendefekt und/oder einem Gaumenspaltendefekt zu haben, um den Homocysteinlevel und/oder -Metabolismus aufrecht zu erhalten und/oder zu normalisieren; Veränderungen der Synthese und/oder dem Funktionieren und/oder den Änderungen von DNS und RNS und den Veränderungen der Zellsynthese; depressiven Erkrankungen.

## Revendications

1. Composé cristallin ou amorphe qui est un folate ou un folate réduit, de D-glucosamine, D-galactosamine, -folate, -dihydrofolate, -tétrahydrofolate, non substitué ou substitué par un fragment de 5-méthyl-, 5-formyl-, 10-formyl-, 5,10-méthylène-, 5,10-méthényl-, le composé, à chaque fois qu'il est envisagé, étant dans une configuration (6R,S), (6S) ou (6R).

2. Composé selon la revendication 1, dans lequel le folate et le folate réduit sont choisis parmi le groupe constitué de folate de D-glucosamine, folate de D-galactosamine, (6R,S)-tétrahydrofolate de D-glucosamine, (6S)-tétrahydrofolate de D-glucosamine, (6R)-tétrahydrofolate de D-glucosamine ; (6R,S)-tétrahydrofolate de D-galactosamine, (6S)-tétrahydrofolate de D-galactosamine, (6R) tétrahydrofolate de D-galactosamine ; 5-méthyl-(6R,S)-tétrahydrofolate de D-glucosamine, 5-méthyl-(6S)-tétrahydrofolate de D-glucosamine, 5-méthyl-(6R)-tétrahydrofolate de D-glucosamine ; 5-méthyl-(6R,S)-tétrahydrofolate de D-galactosamine, 5-méthyl-(6S)-tétrahydrofolate de D-galactosamine, 5-méthyl-(6R)-tétrahydrofolate de D-galactosamine.

3. Composé selon la revendication 1 ou 2, dans lequel le folate et le folate réduit sont choisis parmi le groupe constitué de 5-méthyl-(6R,S)-tétrahydrofolate de D-glucosamine, 5-méthyl-(6S)-tétrahydrofolate de D-glucosamine, folate de D-glucosamine, 5-méthyl-(6R,S)-tétrahydrofolate de D-glucosamine, 5-méthyl-(6S)-tétrahydrofolate de D-glucosamine, folate de D-glucosamine, 5-méthyl-(6R,S)-tétrahydrofolate de D-glucosamine, 5-méthyl-(6S)-tétrahydrofolate de D-glucosamine, folate de D-galactosamine.

4. Composé selon l'une quelconque des revendications précédentes, qui est du 5-méthyl-(6R,S)-tétrahydrofolate de D-glucosamine ou du 5-méthyl-(6S) - tétrahydrofolate de D-glucosamine.

5. Composé selon l'une quelconque des revendications 1 à 4, qui est du folate de D-glucosamine.

6. Composé selon l'une quelconque des revendications précédentes, dans lequel le folate réduit est dans une configuration (6S).

7. Composé selon l'une quelconque des revendications précédentes, qui est dans un état amorphe.

8. Composé selon l'une quelconque des revendications précédentes, dans lequel le folate réduit est du 5-méthyl-(6S)-tétrahydrofolate de D-glucosamine ou du 5-méthyl-(6S)-tétrahydrofolate de D-galactosamine.

9. Composé selon l'une quelconque des revendications précédentes, qui est 5-méthyl-(6S)-tétrahydrofolate de D-glucosamine.

10. Composition comprenant au moins un composé selon l'une quelconque des revendications précédentes.

11. Composition selon la revendication précédente, dans laquelle le composé est un composé cristallin ou amorphe qui est un folate ou un folate réduit de D-glucosamine et D-galactosamine et dans laquelle elle comprend en outre au moins une des substances suivantes : du monohydrate de lactose, une cellulose microcristalline, du glycolate d'amidon sodique, de l'acide stéarique, des vitamines, de l'acide ascorbique, des ascorbates, de l'arginine, de la lysine, de la thiamine, des acides gras ω-3 et/ou ω-6 essentiels saturés ou insaturés, SAMe, de la cobalamine, de l'ubiquinone, des probiotiques, des phospholipides, de la sérine, de la choline, de l'inositol, de l'éthylènediamine, des extraits botaniques, de la mélatonine, des minéraux, des oligo-éléments.

12. Composition selon la revendication 10 ou 11, dans laquelle le folate et le folate réduit sont choisis parmi le groupe constitué de folate de D-glucosamine, folate de D-galactosamine, (6R,S)-tétrahydrofolate de D-glucosamine, (6S)-tétrahydrofolate de D-glucosamine, (6R)-tétrahydrofolate de D-glucosamine ; (6R,S)-tétrahydrofolate de D-galactosamine, (6S)-tétrahydrofolate de D-galactosamine, (6R)-tétrahydrofolate de D-galactosamine ; 5-méthyl-(6R,S)-tétrahydrofolate de D-glucosamine, 5-méthyl-(6S) -tétrahydrofolate de D-glucosamine, 5-méthyl-(6R)-tétrahydrofolate de D-glucosamine ; 5-méthyl-(6R,S)-tétrahydrofolate de D-galactosamine, 5-méthyl-(6S)-tétrahydrofolate de D-galactosamine, 5-méthyl-(6R)-tétrahydrofolate de D-galactosamine.

13. Composition selon l'une quelconque des revendications 10 à 12, dans laquelle le folate et le folate réduit sont choisis parmi le groupe constitué de 5-méthyl-(6R,S)-tétrahydrofolate de D-glucosamine, 5 méthyl-(6S)-tétrahydrofolate de D-glucosamine, folate de D-glucosamine, 5-méthyl-(6R,S)-tétrahydrofolate de D-galactosamine, 5-méthyl-(6S)-tétrahydrofolate de D-galactosamine, folate de D-galactosamine.

14. Composition selon l'une quelconque des revendications 10 à 13, dans laquelle le composé est du 5-méthyl-(6R,S)-tétrahydrofolate de D-glucosamine ou du 5-méthyl-(S)-tétrahydrofolate de D-glucosamine.

15. Composition selon l'une quelconque des revendications 10 à 14, dans laquelle le composé est du folate de D-glucosamine.

16. Composition selon l'une quelconque des revendications 10 à 15, dans laquelle les vitamines, les acides gras, les probiotiques et les extraits botaniques sont choisis -respectivement- parmi les groupes suivants constitués de : la vitamine A, B, C, D, E, K, PP ; DHA, ARA, EPA ; de lactobacilles, spores, levures ; de myrtille, leucocyanidines, ginkgo biloba, ginseng, thé vert, valériane, passiflore, camomille.

17. Composition selon l'une quelconque des revendications 10 à 16, dans laquelle la vitamine B est soit la vitamine B1, B2, B6 ou B12 et la vitamine D est la vitamine D3.

18. Utilisation d'au moins un composé et/ou d'une composition selon l'une quelconque des revendications précédentes, pour la préparation d'un médicament, un additif alimentaire ou un complément nutritionnel, pour la prévention et/ou le traitement de déficiences ou de maladies, affectées positivement par l'administration à la fois de folates et de folates réduits, choisies parmi une affection neurologique telles que, par exemple, une encéphalite subaigüe associée à de la démence ou des myélopathies vacuolaires ; pathopsychologique, vasculaire et cardiovasculaire telles que, par exemple, une maladie artérielle occlusive prématurée, une maladie vasculaire sévère dans la petite enfance et l'enfance, une sténose artérielle progressive, une claudication intermittente, une hypertension réno-vasculaire, une maladie cérébro-vasculaire ischémique, une occlusion prématurée d'artère rétinienne et de veine rétinienne, une maladie artérielle occlusive cérébrale, une mort prématurée due à une maladie thromboembolique et/ou une maladie du coeur ischémique ; des maladies auto-immunes, telles que, par exemple, du psoriasis, une maladie coeliaque, des états arthritiques et d'inflammation ; une anémie mégaloblastique due à une déficience en polate, une mauvaise absorption intestinale, pour réduire le risque qu'une femme fasse une fausse couche et/ou ait un foetus ayant une anomalie du tube neural, une anomalie de bec de lièvre, et/ou une anomalie de fente du palais, pour maintenir et/ou normaliser le niveau d'homocystéine et/ou le métabolisme ; des altérations de la synthèse et/ou du fonctionnement et/ou des changements de l'ADN ou l'ARN et les altérations de la synthèse cellulaire ; des maladies dépressives.
